# EUROPÄISCHE PATENTSCHRIFT

(11) **EP 0 751 763 B1**
(45) Veröffentlichungstag und Bekanntmachung des Hinweises auf die Patenterteilung: **24.10.2001**
(21) Anmeldenummer: 95915157.2
(22) Anmeldetag: 24.03.1995
(51) Int. Cl.: A61K 7/48

(54) **GEGEN HAUTALTERUNG WIRKSAME STOFFE ODER WIRKSTOFFKOMBINATIONEN UND ZUBEREITUNGEN**
SUBSTANCES, COMBINATIONS OF ACTIVE SUBSTANCES AND COMPOSITIONS AGAINST SKIN AGEING
SUBSTANCES, COMBINAISONS DE SUBSTANCES ACTIVES ET COMPOSITIONS EFFICACES CONTRE LE VIEILLISSEMENT DE LA PEAU

(30) Priorität: 25.03.1994 DE 4410238
(43) Veröffentlichungstag der Anmeldung: 08.01.1997
(73) Patentinhaber: Beiersdorf Aktiengesellschaft, 20245 Hamburg (DE)
(72) Erfinder: HOPPE, Udo, 22397 Hamburg (DE); SAUERMANN, Gerhard, 24649 Wiemersdorf (DE); SCHREINER, Volker, 20259 Hamburg (DE); STEIGER, Klaus-Michael, 8700 Küsnacht (Zürichsee) (CH)
(86) Internationale Anmeldenummer: EP9501117
(87) Internationale Veröffentlichungsnummer: WO9526181

(56) Entgegenhaltungen:
- WO-A-88/03015

## Beschreibung

Die vorliegende Erfindung betrifft Wirkstoffe und Zubereitungen zur Pflege gealterter Haut sowie zur Prophylaxe und Behandlung der chronologischen Hautalterung, die zusätzlich durch exogene Faktoren verstärkt oder beschleunigt sein kann.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Couperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Weiterhin sind kosmetische Zubereitungen mit Coenzym Q-10 aus der DE-A-33 09 850 bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

In der japanischen Offenlegungsschrift 58,180,410 ist die Eignung von Coenzym Q-10 für Kosmetika beschrieben. Es soll den Hautzellmetabolismus aktivieren und die Oxidation unterdrücken. Coenzym Q10 hat im Resultat eine wichtige Funktion bei der Prävention von Hautschäden durch UV-Strahlen und der Prävention von Hautalterung. Bei 20 bis 40-jährigen wird die Hautrauhigkeit gebessert, indem der Haut Feuchtigkeit gegeben wird.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Diese Aufgaben werden durch die Erfindung gelöst.

Gegenstand der Erfindung sind topische Zubereitungen mit einem Gehalt an einer Verbindung oder mehreren Verbindungen aus der Gruppe der Ubichinone und deren Derivaten in Kombination mit einem Gehalt an einer Verbindung oder mehreren Verbindungen aus der Gruppe der Plastochinone und deren Derivaten oder Zubereitungen mit einem Gehalt an einer Verbindung oder mehreren Verbindungen aus der Gruppe der Plastochinone und deren Derivaten (als nur einer Wirkstoffgruppe).

Die erfindungsgemäßen topischen Zubereitungen können kosmetische oder dermatologische Zubereitungen sein. Sie dienen, wie auch die Wirkstoffe, vorzugsweise zur Prophylaxe und/oder Behandlung der chronologischen und/oder exogen bedingten Hautalterung.

Gegenstand der Erfindung ist auch die Verwendung einer Verbindung oder mehrerer Verbindungen aus der Gruppe der Ubichinone und deren Derivate in Kombination mit einer Verbindung oder mehreren Verbindungen aus der Gruppe der Plastochinone und deren Derivate oder die Verwendung einer Verbindung oder mehreren Verbindungen aus der Gruppe der Plastochinone und deren Derivaten, jeweils zur Prophylaxe und/oder Behandlung der chronologischen und/oder exogen bedingten Hautalterung.

Mit "Ubichinone" und "Plastochinone" sind hier auch "Ubichinone und deren Derivate" und "Plastochinone und deren Derivate" gemeint.

Es hat sich überraschenderweise gezeigt, daß Ubichinone und deren Derivate und/oder Plastochinone und deren Derivate nicht nur die Haut vor Schäden durch chronologische Hautalterung, insbesondere aber Lichtalterung schützen, sondern auch die Reparatur von Schäden herbeiführen, was in signifikanter Weise Nachteilen des Standes der Technik abhilft. Diese Wirkung dieser Stoffgruppe auf strukturelle Veränderungen der Altershaut ist besonders vorteilhaft.

Bei der chronologischen Hautalterung kommt es alterungsbedingt insbesondere zu folgenden Strukturschäden und Funktionsstörungen:
a) Trockenheit, Rauhigkeit und Ausbildung von Hautfältchen,
b) Juckreiz
c) verminderte Rückfettung durch Talgdrüsen (z.B. nach dem Waschen)
   und den Störungen bei seniler Xerosis, insbesondere solchen, wie unter a) bis c) beschrieben.
   Bei der exogen bedingten Hautalterung, die z.B. durch UV-Licht und chemische Noxen bewirkt wird, kommt es insbesondere zu den folgenden Störungen:
d) Sichtbare Gefäßerweiterungen (Couperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die Ubichinone sind aus der Literatur bekannt (z.B. "Römpp Chemie Lexikon", Georg Thieme Verlag Stuttgart, New York, 9. Auflage, S. 4784-4785 oder "The Merck Index", 11th Edition, Merck & Co., Inc. Rahway, N.Y., USA, Abstr. 9751 (1989). Sie werden auch als Mitochinone oder Coenzyme Q bezeichnet. Die Anzahl der Isopren-Einheiten in der Seitenkette wird mit n in der Bezeichnung Coenzyme Q-n angegeben, worin n eine ganze Zahl bedeutet. Bevorzugt werden Ubichinone oder Coenzyme Q-n mit n=0-12, besonders bevorzugt n=1-12 und insbesondere n=6 bis 10. Gegenstand der Erfindung ist also auch der Chinongrundkörper des Ubichinons ohne Isoprensubstituenten. Erfindungsgemäße Ubichinone oder deren Derivate sind z.B. auch Alkyl-Ubichinone, insbesondere 6-Alkyl-Ubichinone mit vorzugsweise C₁-C₁₂-Alkyl-Resten. Bevorzugt wird Decyl-Ubichinon, insbesondere 6-Decyl-Ubichinon oder 2,3-Dimethoxy-5-methyl-6-decyl-1,4-benzochinon.

Die Plastochinone sind ebenfalls aus der Literatur bekannt (z.B. "Römpp Chemie Lexikon", Georg Thieme Verlag, Stuttgart, New York, 9. Auflage, S. 3477). Sie sind in der Struktur eng mit den Ubichinonen verwandt und zählen auch zu den Isoprenoid-Chinonen, da sie am Chinonring eine Seitenkette aus Isopren-Einheiten tragen. Bevorzugt werden Plastochinone mit 0 - 12, besonders bevorzugt 1 - 10 und insbesondere 6 bis 10 Isopren-Einheiten in der Seitenkette. Gegenstand der Erfindung ist also auch der Chinongrundkörper des Plastochinons ohne Isoprensubstituenten. Erfindungsgemäße Plastochinone oder deren Derivate sind z.B. auch Alkyl-Plastochinone mit vorzugsweise C₁-C₁₂-Alkyl-Resten. Bevorzugt werden Decyl-Plastochinone, insbesondere 5- oder 6-Decyl-Plastochinon oder 2,3-Dimethyl-5-decyl-1,4-benzochinon.

Ubichinone fungieren bei der biologischen, mitochondralen Oxidation als Elektronenüberträger und spielen so eine bedeutende Rolle beim Energiestoffwechsel der tierischen Zellen. In kosmetischen Zubereitungen finden Ubichinone seit langem Anwendung als Antioxidantien zum Schutz oxidationempfindlicher Substanzen.

Plastochinone sind analoge Verbindungen aus dem Pflanzenbereich, die bei der Photosynthese in den Chloroplasten der pflanzlichen Zellen eine Rolle spielen. Sie unterscheiden sich von den Ubichinonen in drei Substituenten am Chinonring, wobei die zwei Methoxy-Gruppen in den Ubichinonen durch Methylgruppen und eine Methylgruppe durch ein Wasserstoffatom ersetzt sind. Strukturgleich sind jedoch die kettenförmig gebundenen Isopren-Einheiten (vergl. z.B. Pfister und Arntzen, Z. für Naturforschung C34; 996ff, 1979).

Besonders bevorzugt werden die folgenden erfindungsgemäßen Wirkstoffe und Kombinationen damit:
Coenzym Q - 10, Coenzym Q - 9, Coenzym Q - 8, Coenzym Q - 7, Coenzym Q - 6,

Plastochinon mit 10 Isopreneinheiten (auch PQ-10 genannt entsprechend der IUB-Abkürzung PQ für Plastochinone, in der Formel PQ-n soll n die Anzahl der Isopren-Einheiten (0 bis 12) angeben), PQ-9, PQ-8, PQ-7, PQ-6.

Die erfindungsgemäßen Wirkstoffkombinationen oder Wirkstoffe können in den topischen Zubereitungen in Mengen von 0,001 bis 99 Gew.-%, z.B. auch in Mengen von 0,001 bis 50 Gew.-% vorliegen, jeweils bezogen auf das Gesamtgewicht der Zubereitungen.

Vorzugsweise können die erfindungsgemäßen Wirkstoffkombinationen oder Wirkstoffe in den topischen Zubereitungen in Mengen von 0,01 bis 10 Gew.-%, insbesondere in Mengen von 0,1 bis 1 Gew.-% vorliegen, jeweils bezogen auf das Gesamtgewicht der Zubereitungen.

In den Ubichinon/Plastochinon-Kombinationen können die Gewichtsverhältnisse der beiden Komponenten in großen Bereichen schwanken, z.B. im Verhältnis 1 : 100 bis 100 : 1, vorzugsweise im Verhältnis 1 : 10 bis 10 : 1. Sie können z.B. auch im Gewichtsverhältnis 1 : 2 bis 2 : 1 oder 1 : 1 vorliegen.

Ganz besonders bevorzugt enthalten die Hautpflegeprodukte oder Dermatika 0,2 bis 0,4 Gew.-%, insbesondere 0,3 Gew.-% Coenzym Q-10 in Kombination mit einem oder mehreren Plastochinonen oder deren Derivaten.

Im Rahmen der Anmeldung sind stets Gewichtsprozente bezogen auf 100% Gesamtzusammensetzung des jeweiligen erfindungsgemäßen Hautpflegepräparates oder Dermatikums gemeint.

Erfindungsgemäße topische Zubereitungen oder Zusammensetzungen mit den erfindungsgemäßen Kombinationen und Wirkstoffen sind alle gängigen Anwendungsformen, z.B. Cremes (W/O, O/W, W/O/W), Gele, Lotionen, Milchen.

Die erfindungsgemäßen topischen Zubereitungen können als flüssige, pastöse oder feste Zubereitungen formuliert werden, beispielsweise als wäßrige oder alkoholische Lösungen, wäßrige Suspensionen, Emulsionen, Salben, Cremes, Öle, Pulver oder Stifte. In Abhängigkeit von der gewünschten Formulierung können die Wirkstoffe in pharmazeutische und kosmetische Grundlagen für topische Applikationen eingearbeitet werden, die als weitere Komponenten beispielsweise Ölkomponenten, Fett und Wachse, Emulgatoren, anionische, kationische, ampholyitsche, zwitterionische und/oder nichtionogene Tenside, niedere ein- und mehrwertige Alkohole, Wasser, Konservierungsmittel, Puffersubstanzen, Verdickungsmittel, Duftstoffe, Farbstoffe und Trübungsmittel enthalten. Vorteilhaft können die erfindungsgemäßen Wirkstoffe auch in transdermalen therapeutischen Systemen, insbesondere kubischen Systemen verwendet werden.

Es ist ferner von Vorteil, den Zubereitungen Antioxidantien (z.B. alpha-Tocopherol, Vitamin E und C, Imidazole, alpha-Hydroxycarbonsäuren (z.B. Äpfelsäure, Glycolsäure, Gluconsäure, Salicylsäure sowie deren Derivate) und/oder Eisenkomplexbildner (z.B. EDTA, alpha-Hydroxyfettsäuren) und/oder bekannte UV-Lichtschutzfilter in Mengen von z.B. 0,1 bis 10 Gewichtsprozenten zuzusetzen, um die Stabilität der oxidationsempfindlichen Ubichinone oder Plastochinone zu gewährleisten.

Auch ist es vorteilhaft, den Zubereitungen insbesondere 0,01 - 10 Gewichtsprozente an Stoffen bzw. Stoffkombinationen des aeroben zellulären Energiestoffwechsels (z.B. zelluläre Energieüberträger (wie Kreatin, Guanin, Guanosin, Adenin, Adenosin, Nicotin, Nicotinamid, Riboflavin), Coenzyme (z.B. Pantothensäure, Panthenol, Liponsäure, Niacin), Hilfsfaktoren (z.B. L-Carnitin, Uridin), Substrate (z.B. Hexosen, Pentosen, Fettsäuren) und intermediäre Stoffwechselprodukte (z.B. Zitronensäure, Pyruvat) und/oder Glutathion zuzusetzen.

Vorteilhaft können erfindungsgemäße Zubereitungen außerdem Substanzen enthalten, die UV-Strahlung im UVA- und/oder im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel für die Haut dienen. In den Zubereitungen wirken die UV-Absorber gegenüber den Wirkstoffen als Antioxidantien.

Enthalten die erfindunsgemäßen Emulsionen UVB-Filtersubstanzen, können diese öllöslich oder wasserlöslich sein. Erfindungsgemäß vorteilhafte öllösliche UVB-Filter sind z.B.:
3-Benzylidencampher-Derivate, vorzugsweise 3-(4-Methylbenzyliden)campher, 3-Benzylidencampher.

Vorteilhaft wasserlösliche UVB-Filter sind z.B.:
Salze der 2-Phenylbenzimidazol-5-sulfonsäure wie ihr Natrium-, Kalium- oder ihr Triethanolammonium-Salz, sowie die Sulfonsäure selbst.

Es kann auch von Vorteil sein, erfindungsgemäße Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'isopropylphenyl)propan-1,3dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung sind also auch die Kombinationen der erfindungsgemäßen Wirkstoffe, insbesondere in den topischen Zubereitungen, mit Antioxydantien, Stoffen des aeroben zellulären Energiestoffwechsels und/oder UV-Absorbern, durch die sich z.B. die Stabilität und die Wirkung der Zubereitung verbessern läßt.

Die vorstehend aufgeführten Beispiele für kombinierbare Wirkstoffe aus den angegebenen Wirkstoffgruppen dienen dazu, die Erfindung zu beschreiben, ohne daß beabsichtigt ist, die Erfindung auf diese Beispiele zu beschränken.

Darüber hinaus können schützende Formulierungsformen angewendet werden, wobei die erfindungsgemäßen Stoffe z.B. in Liposomen, Micellen, Nanosphären usw. aus z.B. hydrierten Amphiphilen, wie z.B. Ceramiden, Fettsäuren, Sphingomyelin und Phosphoglyceriden bzw. in Zyklodextrane eingeschossen (verkapselt) werden. Weiterer Schutz kann durch die Verwendung von Schutzgas (z.B. N₂, CO₂) bei der Formulierung und die Verwendung gasdichter Verpackungsformen erreicht werden.

Weitere Hilfs- und Zusatzstoffe können wasserbindende Stoffe, Verdicker, Füllstoffe, Parfüm, Farbstoffe, Emulgatoren, Wirkstoffe wie Vitamine, Konservierungsmittel, Wasser und/oder Salze sein.

Bei der Verarbeitung der Ubichinone oder Plastochinone und anderer oxidationsempfindlicher Stoffe sollte die Temperatur nicht über 40°C liegen. Ansonsten sind die üblichen Maßregeln zu beachten, die dem Fachmann bekannt sind.

Die erfindungsgemäßen Stoffgruppen lassen sich so in alle kosmetischen Grundlagen einarbeiten. Grundsätzlich sind allerdings W/O- und O/W-und W/O/W-Emulsionen bevorzugt. Besonders vorteilhaft können erfindungsgemäße Kombinationen in Pflegeprodukte wie beispielsweise O/W-Cremes, W/O-Cremes, O/W-Lotionen, W/O-Lotionen usw. eingesetzt werden.

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischung.

Die folgenden Beispiele dienen dazu, die Erfindung zu beschreiben, ohne daß beabsichtigt ist, die Erfindung auf diese Beispiele zu beschränken.

Die angegebenen Teile sind Gewichtsteile.

### Beispiel I

| Hautcreme vom W/O-Typ | |
|---|---|
| | Gew.-Teile |
| Vaseline DAB 9 | 13 |
| Glycerin DAB 9 | 6,3 |
| Wasser (VES, vollentsalzt) | 34,4 |
| Paraffinöl (Mineralöl 5E,Shell) | 35 |
| Cetearylalkohol/PEG-40 Castor Oil/Natriumcetearylsulfat (Emulgade F, Henkel KGaA) | 2,5 |

Zu der 75°C warmen Fettphase wird die 75°C warme Wasserphase gegeben, verrührt und homogenisiert bis eine gleichmäßig weiße Creme entstanden ist. 0,03 Teile Plastochinon mit 9 Isoprengruppen (PQ-9) werden in 8,5 Teilen Paraffinöl gelöst und zu der auf etwa 40°C abgekühlten Creme gegeben und verrührt, bis eine gleichmäßige hellgelbe Creme entstanden ist.

### Beispiel I hat folgende endgültige Zusammensetzung:

| | Gew.-Teile |
|---|---|
| Vaseline DAB 9 | 13 |
| Glycerin DAB 9 | 6,3 |
| Wasser VES | 34,67 |
| Paraffinöl (Mineralöl 5E,Shell) | 43,5 |
| Cetearylalkohol/PEG-40 Castor Oil/Natriumcetearyl sulfat (Emulgade F, Henkel KGaA) | 2,5 |
| Plastochinon PQ-9 | 0,03 |
| | 100 |

### Beispiel II

| Hautcreme vom W/O-Typ | |
|---|---|
| | Gew.-Teile |
| PEG-1 Glyceryl-Oleostearat + Paraffinwachs | 8 |
| Vaseline DAB | 2,8 |
| Paraffinöl (Mineralöl 5E,Shell) | 9,9 |
| Paraffinwachs/Paraffin | 1,8 |
| Ceresin | 2,2 |
| Octyldodecanol | 10 |
| (Eutanol G, Henkel) Propylenglycol | 1 |
| Glycerin | 1 |
| Magnesiumsulfat | 0,7 |
| Wasser VES | 59,7 |
| Summe Additive (Parfüm, Konservierung, Stabilisation) | 0,8 |

Zu der 75°C warmen Fettphase wird die 75°C warme Wasserphase gegeben, verrührt und homogenisiert bis eine gleichmäßig weiße Creme entstanden ist. 0,36 Teile Coenzym Q10 und 0,04 Teile Plastochinon mit 9 Isoprengruppen, PQ-9, werden in 2 Teilen Paraffinöl gelöst und zu der auf etwa 40°C abgekühlten Creme gegeben und verrührt, bis eine gleichmäßige hellgelbe Creme entstanden ist.

### Beispiel II hat folgende endgültige Zusammensetzung:

| | Gew.-Teile |
|---|---|
| PEG-1 Glyceryl-Oleostearat + Paraffinwachs | 8 |
| Vaseline DAB | 2,8 |
| Paraffinwachs/Paraffin | 1,8 |
| Paraffinöl (Mineralöl 5E,Shell) | 11,9 |
| Ceresin | 2,2 |
| Octyldodecanol | 10 |
| Plastochinon, PQ-9 | 0,04 |
| Coenzym Q10 | 0,36 |
| Propylenglycol | 1 |
| Glycerin | 1 |
| Magnesiumsulfat | 0,7 |
| Wasser VES | 59,4 |
| Summe Additive (Parfüm, Konservierunq, Stabilisation) | 0,8 |
| | 100 |

### Beispiel III

| Hautcreme vom O/W-Typ | |
|---|---|
| | Gew.-Teile |
| Octyldodecanol (Eutanol G, Henkel KGaA) | 9,3 |
| Cetearylalkohol/PEG-40 Castor Oil/Natriumcetearyl sulfat (Emulgade F, Henkel KGaA) | 3,7 |
| Wasser VES | 73,7 |
| Glycerin DAB 9 | 4,6 |
| Paraffinöl (Mineralöl 5E, Shell) | 5,8 |

Zu der 75°C warmen Fettphase wird die 75°C warme Wasserphase gegeben, verrührt und homogenisiert bis eine gleichmäßig weiße Creme entstanden ist. 0,54 Teile Coenzym Q10 und 0,35 Teile Coenzym Q6 und 0,01 Gewichtsteile Plastochinon PQ-9 werden in 2 Teilen Paraffinöl gelöst und zu der auf etwa 40°C abgekühlten Creme gegeben und verrührt, bis eine gleichmäßige hellgelbe Creme entstanden ist.

### Beispiel III hat folgende endgültige Zusammensetzung:

| | Gew.-Teile |
|---|---|
| Octyldodecanol (Eutanol G, Henkel KGaA) | 9,3 |
| Cetearylalkohol/PEG-40 Castor Oil/Natriumcetearyl sulfat (Emulgade F, Henkel KGaA) | 3,7 |
| Wasser VES | 73,7 |
| Glycerin DAB 9 | 4,6 |
| Paraffinöl (Mineralöl 5E, Shell) | 7,8 |
| Plastochinon PQ-9 | 0,01 |
| Coenzym Q6 | 0,35 |
| Coenzym Q₁₀ | 0,54 |
| | 100 |

### Beispiel IV

| O/W-Lotion | Gew.-Teile |
|---|---|
| Steareth-2 | 3 |
| Steareth-21 | 2 |
| Cetearylalkohol/PEG-40 Castor Oil/Natriumcetearyl sulfat (Emulgade F, Henkel KGaA) | 2,5 |
| Paraffinöl (Mineralöl 5E, Shell) | 10,1 |
| Propylenglycol | 1 |
| Glycerin | 1 |
| Wasser VES | 74,3 |
| Summe Additive (Parfüm, Konservierung, Stabilisation) | 0,8 |

Zu der 75°C warmen Fettphase wird die 75°C warme Wasserphase gegeben, verrührt und homogenisiert bis eine gleichmäßig weiße Lotion entstanden ist. 0,2 Teile Coenzym Q10 und 0,2 Teile Plastochinon PQ-9 werden in 4 Teilen Paraffinöl gelöst und zu der auf etwa 40°C abgekühlten Lotion gegeben und verrührt, bis eine gleichmäßige hellgelbe Lotion entstanden ist.

### Beispiel IV hat folgende endgültige Zusammensetzung:

| | Gew.-Teile |
|---|---|
| Steareth-2 | 3 |
| Steareth-21 | 2 |
| Cetearylalkohol/PEG-40 Castor Oil/Natriumcetearyl sulfat (Emulgade F, Henkel KGaA) | 2,5 |
| Paraffinöl (Mineralöl 5E, Shell) | 14,1 |
| Propylenglycol | 1 |
| Coenzym Q6 | 0,2 |
| Plastochinon PQ-9 | 0,2 |
| Glycerin | 1 |
| Wasser VES | 74,3 |
| Summe Additive (Parfüm, Konservierung, Stabilisation) | 0,8 |
| | 100 |

### Beispiel V

| O/W-Lotion | Gew.-Teile |
|---|---|
| Octyldodecanol (Eutanol G, Henkel KGaA) | 5,6 |
| Cetearylalkohol/PEG-40 Castor Oil/Natriumcetearyl sulfat (Emulgade F, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |
| Paraffinöl | |
| (Mineralöl 5E, Shell) | 5,7 |

Zu der 75°C warmen Fettphase wird die 75°C warme Wasserphase gegeben, verrührt und homogenisiert bis eine gleichmäßig weiße Creme entstanden ist, 0,04 Teile Coenzym Q10 und 0,36 Teile Plastochinon PQ-9 werden in 5 Teilen Paraffinöl gelöst und zu der auf etwa 40°C abgekühlten Creme gegeben und verrührt bis eine gleichmäßige hellgelbe Creme entstanden ist.

### Beispiel V hat folgende endgültige Zusammensetzung:

| | Gew.-Teile |
|---|---|
| Octyldodecanol (Eutanol G, Henkel KGaA) | 5,6 |
| Cetearylalkohol/PEG-40 Castor Oil/Natriumcetearyl sulfat (Emulgade F, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |
| Paraffinöl (Mineralöl eE, Shell) | 10,7 |
| Coenzym Q-10 | 0,04 |
| Plastochinon PQ-9 | 0,36 |
| | 100 |

### Beispiel VI

| Hautöl | Gew.-Teile |
|---|---|
| Glyceryltricaprylat (Miglyol 812, Dynamit Nobel) | 22,7 |
| Hexyllaurat (Cetiol A, Henkel KGaA) | 22 |
| Octylstearat (Cetiol 886, Henkel KGaA) | 20 |
| Paraffinöl (Mineralöl 5E, Shell) | 35 |
| PlastochinonPQ-9 | 0,1 |
| Coenzym Q₆ | 0,2 |
| | 100 |

Die Komponenten werden bei 25°C verrührt, bis eine gleichmäßige, klare Mischung entstanden ist.

### Beispiel VII

| **Hautöl** | **Gew.-Teile** |
|---|---|
| Glyceryltricaprylat (Miglyol 812, Dynamit Nobel) | 21 |
| Hexyllaurat (Cetiol A, Henkel KGaA) | 20 |
| Octylstearat (Cetiol 886, Henkel KGaA) | 21,7 |
| Paraffinöl (Mineralöl 5E, Shell) | 37 |
| Plastochinon PQ-9 | 0,3 |
| | 100 |

### (Herstellung wie in Beispiel VI)

### Beispiel VIII

| Hautcreme vom W/O-Typ | **Gew.-Teile** |
|---|---|
| Vaseline DAB 9 | 13 |
| Glycerin DAB 9 | 6,3 |
| Wasser VES | 34,4 |
| Paraffinöl (Mineralöl 5E,Shell) | 35 |
| Cetearylalkohol/PEG-40 Castor Oil/Natriumcetearyl- | |
| sulfat (Emulgade F, Henkel KGaA) | 2,5 |

Zu der 75°C warmen Fettphase wird die 75°C warme Wasserphase gegeben, verrührt und homogenisiert bis eine gleichmäßig weiße Creme entstanden ist. 0,27 Teile Coenzym Q10 und 0,03 Gew.-Teile Plastochinon, PQ- 6 werden in 8,5 Teilen Paraffinöl gelöst und zu der auf etwa 40°C abgekühlten Creme gegeben und verrührt, bis eine gleichmäßige hellgelbe Creme entstanden ist.

### Beispiel VIII hat folgende endgültige Zusammensetzung:

| | **Gew.-Teile** |
|---|---|
| Vaseline DAB 9 | 13 |
| Glycerin DAB 9 | 6,3 |
| Wasser VES | 34,4 |
| Paraffinöl (Mineralöl 5E,Shell) | 43,5 |
| Cetearylalkohol/PEG-40 Castor Oil/Natriumcetearyl sulfat (Emulgade F, Henkel KGaA) | 2,5 |
| Plastochinon, 6 Isopreneinheiten | 0,03 |
| Coenzym Q₁₀ | 0,27 |
| | 100 |

## Patentansprüche

1. Topische Zubereitungen mit einem Gehalt an einer Verbindung oder mehreren Verbindungen aus der Gruppe der Ubichinone und deren Derivaten in Kombination mit einem Gehalt an einer Verbindung oder mehreren Verbindungen aus der Gruppe der Plastochinone und deren Derivaten oder Zubereitungen mit einem Gehalt an einer Verbindung oder mehreren Verbindungen aus der Gruppe der Plastochinone und deren Derivaten.

2. Zubereitungen gemäß Anspruch 1, **dadurch gekennzeichnet, daß** die Ubichinone oder Plastochinone 0 bis 12 Isopreneinheiten und/oder gegebenenfalls Alkyl-Reste besitzen.

3. Zubereitungen gemäß Anspruch 1, **dadurch gekennzeichnet, daß** die Ubichinone oder Plastochinone 9 oder 10 Isopreneinheiten besitzen.

4. Zubereitungen gemäß Anspruch 1, **dadurch gekennzeichnet, daß** sie Antioxidantien, Stoffe des aeroben zellulären Energiestoffwechsels und/oder UV-Absorber enthalten.

5. Zubereitungen gemäß Anspruch 1, **dadurch gekennzeichnet, daß** sie kosmetische oder dermatologische Zubereitungen, insbesondere W/O-, O/W-oder W/O/W-Emulsionen sind.

6. Kosmetische Verwendung einer Verbindung oder mehrerer Verbindungen aus der Gruppe der Ubichinone und deren Derivaten in Kombination mit einer Verbindung oder mehreren Verbindungen aus der Gruppe der Plastochinone und deren Derivaten oder die kosmetische Verwendung einer Verbindung oder mehreren Verbindungen aus der Gruppe der Plastochinone und deren Derivaten, jeweils zur Prophylaxe und/oder Behandlung der chronologischen und/oder exogen bedingten Hautalterung.

## Claims

1. Topical formulations having a content of one compound or several compounds from the group consisting of ubiquinones and derivatives thereof in combination with a content of one compound or several compounds from the group consisting of plastoquinones and derivatives thereof or formulations having a content of one compound or several compounds from the group consisting of plastoquinones and derivatives thereof.

2. Formulations according to Claim 1, **characterized in that** the ubiquinones or plastoquinones have 0 to 12 isoprene units and/or, if appropriate, alkyl radicals.

3. Formulations according to Claim 1, **characterized in that** the ubiquinones or plastoquinones have 9 or 10 isoprene units.

4. Formulations according to Claim 1, **characterized in that** they comprise antioxidants, substances of aerobic cell energy metabolism and/or UV absorbers.

5. Formulations according to Claim 1, **characterized in that** they are cosmetic or dermatological formulations, in particular W/O, O/W or W/O/W emulsions.

6. Cosmetic use of one compound or several compounds from the group consisting of ubiquinones and derivatives thereof in combination with one compound or several compounds from the group consisting of plastoquinones and derivatives thereof, or the cosmetic use of one compound or several compounds from the group consisting of plastoquinones and derivatives thereof, in each case for prophylaxis and/or treatment of chronological aging of the skin and/or aging of the skin of exogenous origin.

## Revendications

1. Préparations topiques ayant une teneur en un composé ou plusieurs composés choisis(s) dans le groupe des ubiquinones et leurs dérivés, en association avec une teneur en un composé ou plusieurs composés choisi(s) dans le groupe des plastoquinones et leurs dérivés, ou préparations ayant une teneur en un composé ou plusieurs composés choisi(s) dans le groupe des plastoquinones et leurs dérivés.

2. Préparations selon la revendication 1,
**caractérisées en ce que** les ubiquinones ou les plastoquinones comportent de 0 à 12 unités isoprène et/ou éventuellement des radicaux alkyle.

3. Préparations selon la revendication 1,
**caractérisées en ce que** les ubiquinones ou les plastoquinones comportent 9 ou 10 unités isoprène.

4. Préparations selon la revendication 1,
**caractérisées en ce qu'**elles contiennent des antioxydants, des substances du métabolisme énergétique cellulaire aérobie et/ou des absorbeurs d'UV.

5. Préparations selon la revendication 1,
**caractérisées en ce qu'**elles sont des préparations cosmétiques ou dermatologiques, en particulier des émulsions E/H, H/E ou E/H/E.

6. Utilisation cosmétique d'un composé ou de plusieurs composés choisis(s) dans le groupe des ubiquinones et leurs dérivés, en association avec un composé ou plusieurs composés choisi(s) dans le groupe des plastoquinones et leurs dérivés, ou utilisation cosmétique d'un composé ou de plusieurs composés choisi(s) dans le groupe des plastoquinones et leurs dérivés, chacune pour la prophylaxie et/ou le traitement du vieillissement de la peau dû au temps et/ou à des facteurs exogènes.
